# EUROPEAN PATENT SPECIFICATION

(11) **EP 1 143 247 B1**
(45) Date of publication and mention of the grant of the patent: **25.08.2004**
(21) Application number: 00970050.1
(22) Date of filing: 25.10.2000
(51) Int. Cl.: G01N 33/543, G01N 33/558

(54) **IMMUNE CHROMATOGRAPHIC TEST PIECE AND CHROMATOGRAPHIC ANALYSIS METHOD**
IMMUNOCHROMATOGRAPHISCHES TESTSTÜCK UND CHROMATOGRAPHISCHE ANALYSENMETHODE
PIECE DE TEST DE CHROMATOGRAPHIE IMMUNITAIRE ET PROCEDE D'ANALYSE CHROMATOGRAPHIQUE

(30) Priority: 25.10.1999 JP 30299799
(43) Date of publication of application: 10.10.2001
(73) Proprietor: MATSUSHITA ELECTRIC INDUSTRIAL CO., LTD., Kadoma-shi, Osaka 571-8501 (JP)
(72) Inventor: TAKAHASHI, Mie, Niihama-shi, Ehime 792-0026 (JP); NADAOKA, Masataka, Iyo-shi, Ehime 799-3113 (JP); TANAKA, Hirotaka, Matsuyama-shi, Ehime 791-1102 (JP); NAKAYAMA, Hiroshi, Hirakata-shi Osaka 573-1114 (JP); SHIGETO, Nobuyuki, Kyotanabe-shi Kyoto 610-0354 (JP); KITAWAKI, Fumihisa, Kadoma-shi Osaka 571-0064 (JP)
(74) Representative: Balsters, Robert
(86) International application number: PCT/JP2000/007447
(87) International publication number: WO 2001/031340

(56) References cited:
- EP-A- 0 279 988
- EP-A- 0 761 821
- EP-A- 0 903 584
- JP-A- 9 072 904
- JP-A- 9 119 932
- JP-A- 10 132 817

## Description

### Technical Field

The present invention relates to an immunochromatographic specimen and a chromatograph analyzing method for performing qualitative or quantitative analysis of a liquid sample utilizing antigen-antibody reaction and, more particularly, to those in which analysis can be performed without preprocessing a colored sample such as blood.

### Background Art

Conventionally, an assay method by immunochromatography utilizing antigen-antibody reaction is universally taken as a method for implementing chemical or clinical examination of a liquid sample, such as water analysis or urinalysis. Generally, it is referred to a method for separating and identifying mixtures in accordance with their components by chromatography. An immunochromatographic specimen includes: a portion for adding a liquid sample; a portion for holding a marker reagent which can migrate by infiltration of the liquid sample as well as comprises a substance being bound specifically to a flowing-down analyte included in the liquid sample; a portion that performs binding reaction of the marker reagent and the analyte; and a portion for absorbing a flowing-down sample. When the liquid sample is added to the chromatographic specimen and it is left as it is, a coloring reaction accompanying coloration or decoloration occurs after a predetermined period of time has passed, whereby qualitative judgment based on visual observation is made possible.

An immunochromatograph analyzing method is one which identifies and detects antigen or antibody utilizing the specificity of antigen-antibody reaction. The immunochromatographic specimen further includes a portion which has fixed antigen or antibody which causes antigen-antibody reaction to a complex of the analyte included in the liquid sample and the marker reagent, as compared with the above-described typical chromatographic specimen. When a liquid sample containing an analyte is added to an immunochromatographic specimen, a marker reagent is dissolved by the liquid sample and is developed in the infiltration direction, whereby a predetermined coloration is generated in a measurement region where antigen or antibody is fixed. Therefore, it can be judged whether or not the specified analyte is included in the liquid sample on the basis of the presence of coloration in the measurement region. Since the analysis result is determined by coloring reaction, it is recorded by the visual observation. In this way, determination of the result of analysis in an immunochromatograph analyzing method is extremely easy, and the method can be applied for analyses of various analytes.

Generally, while the analysis by immunochromatography is qualitative, but quantitative analyzing methods have been developed. In Japanese Published Patent Application Hei.8-240591, there is disclosed a method for quantizing a coloration degree by adding a sample to the immunochromatographic specimen thereby to have reaction and thereafter measuring signals of absorption and reflection at the color-reacted portion on the specimen employing a detecting instrument. Further, in Japanese Published Patent Application Hei.8-334511, there is disclosed a method which, images a color-reacted immunochromatographic specimen by an image sensor, and performs analytic processing of a gradated image of the specimen which has been subjected to image-conversion, thereby to quantitatively measure the coloration degree.

In a typical immunochromatographic specimen, there is no means for mechanically controlling infiltration rate of a liquid sample. Since the infiltration rate cannot be controlled artificially in this way, the infiltration rate is dominated by the permeability of the specimen. When analyzing a liquid sample including cell components such as blood, employing this specimen, the viscosity of the liquid sample and the presence of cell components cause clogging in a reaction layer carrier to prevent the infiltration of the liquid sample. This results in a tremendously longer measurement time, during which desiccation of the liquid sample occurs, thereby causing lacking in reaction precision and in quantitative performance.

Further, in an immunochromatograph analyzing method for measuring the coloration degree employing a visual observation or a detecting instrument, the reading of coloring reaction caused by antigen-antibody reaction is prevented by pigments included in a colored sample, for example, blood pigments (red) of blood components, thereby lowering the sensitivity at quantitative measurement or causing errors in measurement.

In order to solve the above-described problems, when measuring a colored sample, specifically, such as whole blood, a preprocessing of centrifugation or the like has been conventionally required for previously removing pigments included in the sample, that is, blood corpuscle components containing the blood pigments. However, this preprocessing makes the immunochromatograph analyzing method complicated, and further causes an increase in cost and reduction in the usability.

The present invention is made in view of the above-described problems, and has for its object to provide an immunochromatographic specimen and a chromatograph analyzing method which can prevent infiltration of the liquid sample into the specimen from being avoided, which can quantitatively read a coloration degree as the result of antigen-antibody reaction without being affected by the background coming from colored components included in the sample, specifically, the blood pigments, and which further require no preprocessing of centrifugation or the like of the liquid sample to be added, specifically, such as blood.

### Disclosure of the Invention

In order to accomplish the objects, according to an immunochromatographic specimen of Claim 1, in an immunochromatographic specimen used at the qualitative or quantitative analysis of an analyte included in a colored sample, a marker itself or a substance which is produced by the marker or a marker after completion of reaction or a reactant by the marker has a wavelength region including an absorption wavelength which is different from the absorption wavelength of the sample which contains the analyte, or having the same absorption wavelength including an absorption coefficient sufficiently higher than the absorption coefficient of the sample.

Further, according to the immunochromatographic specimen of Claim 2, in the immunochromatographic specimen defined in Claim 1, in the case where the sample is blood, the absorption wavelength specific to coloration generated on the specimen is 580nm or above.

Further, according to the immunochromatographic specimen of Claim 3, the immunochromatographic specimen defined in Claim 1 or 2 includes a region for holding a substance, which destroys cell components included in the sample.

Further, according to the immunochromatographic specimen of Claim 4, in the immunochromatographic specimen defined in Claim 3, the substance which destroys cell components includes inorganic matter, surfactant, or saponins.

Further, according to the immunochromatographic specimen of Claim 5, in the immunochromatographic specimen defined in Claim 4, the inorganic matter contains chloride.

Further, according to the immunochromatographic specimen of Claim 6, in the immunochromatographic specimen defined in Claim 4, the surfactant contains nonpolar surfactant.

Further, according to the immunochromatographic specimen of Claim 7, in the immunochromatographic specimen defined in any one of Claims 1 through 6, a coloration degree is qualitatively or quantitatively analyzed with a spectrophotometer.

Further, according to the immunochromatographic specimen of Claim 8, in the immunochromatographic specimen defined in any one of Claims 1 through 7, the marker includes at least one which is selected from a metal sol, an oxidized metal particle, a non-metal sol, a dye sol, a colored particle, a pigment, or an enzyme.

Further, according to the immunochromatographic specimen of Claim 9, in the immunochromatographic specimen defined in any one of Claims 1 through 8, the analyte contains at least one of plasma protein, bacteria, and virus.

Further, according to a chromatograph analyzing method of Claim 10, in a chromatograph analyzing method using the immunochromatographic specimen, the colored sample containing the analyte is added to the immunochromatographic specimen, a coloration degree on the specimen is measured at an absorption wavelength specific to the coloration, and a marker itself or a substance which is produced by the marker or a marker after completion of reaction or a reactant by the marker has a wavelength region including an absorption wavelength which is different from the absorption wavelength of the sample which contains the analyte, or having the same absorption wavelength including an absorption coefficient sufficiently higher than the absorption coefficient of the sample.

Further, according to a chromatograph analyzing method of Claim 11, in the chromatograph analyzing method defined in Claim 10, in the case where the sample is blood, a signal from the marker in a coloration region is measured at a wavelength of any of 580nm or above, thereby qualitatively or quantitatively analyzing the analyte.

Further, according to the chromatograph analyzing method of Claim 12, in the chromatograph analyzing method defined in Claim 10 or 11, the immunochromatographic specimen includes a region where a substance which destroys cell components in the sample is held.

Further, according to the chromatograph analyzing method of Claim 13, in the chromatograph analyzing method defined in Claim 12, the substance which destroys cell components includes inorganic matter, surfactant, or saponins.

Further, according to the chromatograph analyzing method of Claim 14, in the chromatograph analyzing method defined in Claim 13, the inorganic matter contains chloride.

Further, according to the chromatograph analyzing method of Claim 15, in the chromatograph analyzing method defined in Claims 13, the surfactant contains nonpolar surfactant.

Further, according to the chromatograph analyzing method of Claim 16, in the chromatograph analyzing method defined in any one of Claims 10 through 15, a coloration degree is qualitatively or quantitatively analyzed with a spectrophotometer.

Further, according to the chromatograph analyzing method of Claim 17, in the chromatograph analyzing method defined in any one of Claims 10 through 16, the marker includes at least one which is selected from a metal sol, an oxidized metal particle, a non-metal sol, a dye sol, a colored particle, a pigment, or an enzyme.

Further, according to the chromatograph analyzing method of Claim 18, in the chromatograph analyzing method defined in any one of Claims 10 through 17, the analyte contains at least one of plasma protein, bacteria, and virus.

According to an immunochromatographic specimen of Claims 1, 2, 8 and 9, in an immunochromatographic specimen used at the qualitative or quantitative analysis of an analyte included in a colored sample, a marker itself or a substance produced by the marker has a wavelength region including an absorption wavelength which is different from the absorption wavelength of the sample containing the analyte, or having the same absorption wavelength including an absorption coefficient which is sufficiently higher than the absorption coefficient of the sample. Therefore, in the analysis of the sample containing colored components, the qualitative or quantitative analysis of the coloration degree can be performed without being affected by the background coming from the colored components, thereby realizing a chromatograph analysis with high sensitivity and high performance.

According to the immunochromatographic specimen of Claims 3 through 6, the immunochromatographic specimen defined in Claim 1 or 2 comprises a region for holding a substance which destroys cell components included in the sample. Therefore, besides the above-described effects of Claim 1 or 2, the cell components are destroyed so as to smooth infiltration of the sample into the specimen at the time of analysis of the sample containing the cell components, the measurement time is shortened, and the quantitative analysis can be performed more exactly. Further, the preprocessing of centrifugation or the like of the sample having the cell components such as blood becomes unnecessary, thereby realizing the lowering of cost and the improvement in usability.

According to the immunochromatographic specimen of Claim 7, in the immunochromatographic specimen defined in any one of Claims 1 through 6, a coloration degree is qualitatively or quantitatively analyzed with a spectrophotometer. Therefore, besides the above-described effects of Claims 1 through 6, when a reflecting spectrophotometer is used, an opaque material can be used as a support which supports a reaction layer, and further the measurement can be performed without being cautious of the rear surface state of the reaction layer. Further, when a permeable spectrophotometer is used, the coloration degree of a deep portion in the reaction layer can be also measured, thereby performing qualitative or quantitative analysis with high sensitivity and high performance.

According to a chromatograph analyzing method of Claims 10, 11, 17 and 18, in a chromatograph analyzing method using the immunochromatographic specimen, the colored sample containing the analyte is added to the immunochromatographic specimen, a coloration degree on the specimen is measured at an absorption wavelength specific to the coloration, and a marker itself or a substance produced by the marker has a wavelength region including an absorption wavelength which is different from the absorption wavelength of the sample containing the analyte, or having the same absorption wavelength including an absorption coefficient which is sufficiently higher than the absorption coefficient of the sample. Therefore, in the analysis of the sample containing colored components, the qualitative or quantitative analysis of the coloration degree can be performed without being affected by the background coming from the colored components, thereby realizing a chromatograph analysis with high sensitivity and high performance.

According to the chromatograph analyzing method of Claims 12 through 15, the chromatograph analyzing method defined in Claim 10 or 11 comprises a region for holding a substance which destroys cell components included in the sample. Therefore, besides the above-described effects of Claim 10 or 11, the cell components are destroyed so as to smooth infiltration of the sample into the specimen at the time of analysis of the sample containing the cell components, the measurement time is shortened, and the quantitative analysis can be performed more exactly. Further, the preprocessing of centrifugation or the like of the sample having the cell components such as blood becomes unnecessary, thereby realizing the lowering of cost and the improvement in usability.

According to the chromatograph analyzing method of Claim 16, in the chromatograph analyzing method defined in any one of Claims 10 through 15, a coloration degree is qualitatively or quantitatively analyzed with a spectrophotometer. Therefore, besides the above-described effects of Claims 10 through 15, when a reflecting spectrophotometer is used, an opaque material can be used as a support which supports a reaction layer, and further the measurement can be performed without being cautious of the rear surface state of the reaction layer. Further, when a permeable spectrophotometer is used, the coloration degree of a deep portion in the reaction layer can be also measured. Therefore, qualitative or quantitative analysis can be performed with high sensitivity and high performance.

### Brief Description of the Drawings

Figure 1 is a diagram illustrating an immunochromatographic specimen according to a first embodiment of the present invention.
Figure 2 is a diagram illustrating the immunochromatographic specimen according to a second embodiment of the present invention.
Figures 3 are diagrams illustrating quantitative performances according to an example of the present invention in the case (a) of measuring a coloration region at the wavelength of 520nm and in the case (b) of measuring the same at the wavelength of 610nm, respectively.

### Best Mode for Carrying out the Invention

### Embodiment 1.

Hereinafter, an immunochromatographic specimen and a chromatograph analyzing method according to the first embodiment of the present invention will be described with reference to the drawings.

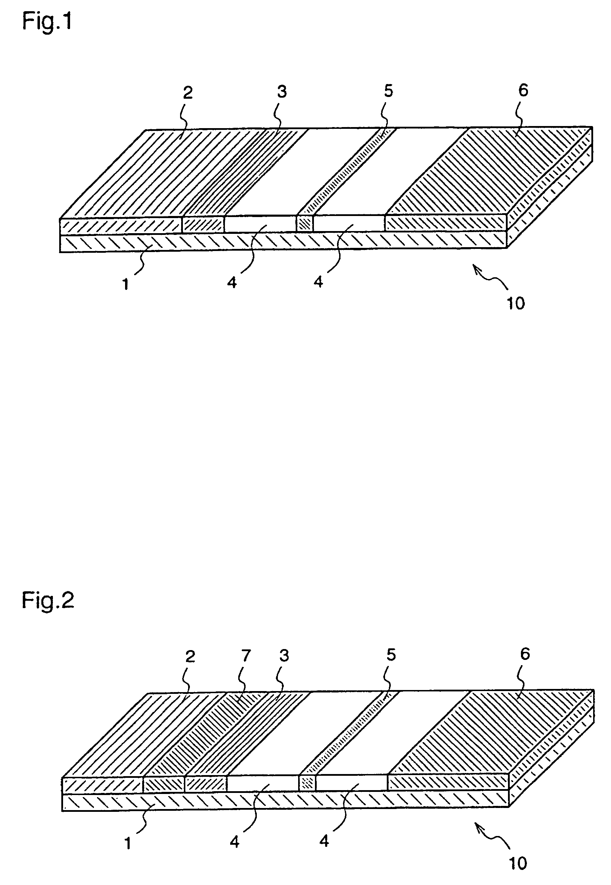

Figure 1 is a diagram illustrating an immunochromatographic specimen 10 according to the first embodiment of the present invention.

In figure 1, the immunochromatographic specimen 10 includes a reaction layer carrier support 1, a sample addition portion 2, a marker holding portion 3, a reaction layer 4, a specific protein fixing portion 5, and an absorption portion 6.

The reaction layer carrier support 1 is composed of liquid-impermeable plastic or the like and supports chromatographic materials. The sample addition portion 2 is composed of an absorbent material such as non-woven fabric, and adds or applies a liquid sample. The marker holding portion 3 holds a marker reagent so as to dissolve into the non-woven fabric or the like. The reaction layer 4 is composed of nitrocellulose or the like. The specific protein fixing portion 5 fixes specific protein which performs binding-reaction specifically to an analyte such as antigen or antibody in accordance with the reaction mode, onto the region of the reaction layer 4. The absorption portion 6 finally absorbs the liquid sample. The sample addition portion 2, the marker holding portion 3, the reaction layer 4, the specific protein fixing portion 5, and the absorption portion 6 as described above are respectively formed above the reaction layer carrier support 1. It is necessary to select an appropriate marker reagent and an appropriate specific protein, in accordance with the analyzed sample and the analyte.

Further, a substance produced by the marker is a substance which, for example, is produced by the marker in the specific protein fixing portion 5, and can be luminous. A marker after completion of reaction is a marker after coloration, for example, by coloring reaction. A reactant by the marker is, for example, a substance obtained after being colored by adding a substrate to the specific protein fixing portion 5 where the marker is fixed or the like, in a case where the marker is an enzyme. A dye sol, a water-soluble pigment, a metal sol such as a gold colloid, a non-metal sol such as selenium and carbon, an oxidized metal particle such as ferrite, a colored particle such as latex, an enzyme or the like, or a complex of these substances and a substance which is bound specifically to the analyte is used as the marker reagent which is held by the marker holding portion 3.

Further, a structure of the immunochromatographic specimen 10 in figure 1 is an example. Any structure in which chromatography is performed may be taken, and the used members may be composed of arbitrary carrier and reagent.

A specimen comprising a chromatographic material composed of an arbitrary porous carrier, such as nitrocellulose and glassfiber filter, is used as the immunochromatographic specimen 10.

Next, the immunochromatographic specimen 10 and a chromatograph analyzing method according to the first embodiment will be described.

In figure 1, when the liquid sample is added to the sample addition portion 2, the liquid sample infiltrates into the sample addition portion 2 and reaches the marker holding portion 3. Next, the marker reagent held in the region of the marker holding portion 3 is dissolved by infiltration of the liquid sample and infiltrates into the reaction layer 4 together with the liquid sample. There is provided the specific protein fixing portion 5 to which the specific protein is fixed on the region of the reaction layer 4. When the liquid sample contains the analyte, the specific protein performs antigen-antibody reaction to the complex of the analyte and the marker reagent, and a kind of coloring reaction is observed at the region of the specific protein fixing portion 5. When the liquid sample does not contain the analyte, no antigen-antibody reaction occurs, and no coloring reaction is observed, either. Finally, the liquid sample is absorbed at the absorption portion 6, and thereby concluding the reaction.

After concluding the above-described reaction, a signal from the marker in the coloration region is measured by employing an arbitrary detecting instrument. When the sample is whole blood, the measurement is preferably performed, for example, at an arbitrary wavelength of 580nm or above. Thereby, the presence of coloration, and the coloration degree can be qualitatively or quantitatively analyzed. It can be detected whether or not the liquid sample contains the analyte by the above-described analysis, and, when the liquid sample contains the analyte, its quantity can be measured. Further, the "signal from the marker" described above concretely means a color of the marker which itself is an colored item, light emission by UV irradiation or voltage application, or coloration by coloring reaction.

In the measurement of the signal from the marker in the coloration region, besides a case where an integral value of an absorbance or an absorbance distribution is measured by an arbitrary wavelength, the absorbance may be measured in the wavelength region having an arbitrary width. In this case, the absorbance can be integrated in the specified wavelength region and be subjected to quantitative analysis.

As described above, according to the immunochromatographic specimen and the chromatograph analyzing method of the first embodiment, the signal from the marker in the coloration region on the immunochromatographic specimen 10 is measured, for example, at an arbitrary wavelength of 580nm or above, whereby in the analysis of the sample containing colored components, more particularly, blood pigments in blood, measurement of the coloration degree is performed at the wavelength except 200-580nm, which is an absorption wavelength of blood. Therefore, the qualitative or quantitative measurement of the coloration degree can be performed without being affected by the background coming from colored components included in the liquid sample, particularly, blood pigments, thereby reducing the measurement error, and realizing the qualitative or quantitative analysis by the immunochromatography with higher sensitivity and higher performance. Further, a chromatograph qualitative analysis by visual observation of the colored sample can be realized in accordance with the used marker reagent.

Further, in this first embodiment, sandwich reactions having the measurement mode of the binding reaction between the marker complex and the analyte and the binding reaction between this reactant and a fixed substance is described as an example. However, the measurement can be performed even as a competition reaction in which the analyte and the marker complex competitively reacts to the fixed substance.

Further, in this first embodiment, in a case where the sample is whole blood, the signal from the marker is measured at an arbitrary wavelength of 580nm or above. However, the measurement may be preferably performed at an arbitrary wavelength from 580nm to 700nm.

Further, in the chromatograph analyzing method of the first embodiment, for example, a reflecting spectrophotometer can be used as the detecting instrument for measuring the coloration degree of the immunochromatographic specimen 10. In addition to the similar effects as the first embodiment, an opaque material can be used as the reaction layer carrier support 1 which supports the reaction layer 4. Therefore, choices of materials are increased, and further the measurement can be performed without being cautious of the rear surface state of the reaction layer 4.

Further, in the chromatograph analyzing method of the first embodiment, for example, a permeable spectrophotometer can be used as the detecting instrument for measuring the coloration degree of the immunochromatographic specimen 10. In addition to the similar effects as the first embodiment, the coloration degree of a deep portion in the reaction layer 4 can be also measured. Therefore, qualitative or quantitative analysis is possible with higher sensitivity and higher performance. Further, the marker reagent with low concentration can be used.

In this first embodiment, blood is especially used as the sample containing the colored components. However, this is just an example. The sample containing colored components except blood can be analyzed. For example, urine, fecal occult blood, crushed liquid from foods or the like are also used. In this case, it is necessary to select the marker reagent and the specific protein in accordance with the analyzed sample and the analyte. That is, it is necessary that an absorption coefficient at the wavelength specific to the color reaction is sufficiently higher than the absorption coefficient of the sample at the same wavelength. Further, in the measurement of coloration degree by the detecting instrument, the measurement may be performed at the wavelength specific to the coloring reaction. Further, the "specific wavelength" described above means an arbitrary wavelength or a broad wavelength region having an arbitrary width, except a maximum wavelength region of the colored sample. Further, the "sufficiently higher" described above means that the signal from the marker by the color reaction is high to the extent that a reliable result of the qualitative or quantitative analysis is obtained. Embodiment 2.

Hereinafter, an immunochromatographic specimen and a chromatograph analyzing method according to a second embodiment of the present invention will be described with reference to the drawings.

Figure 2 is a diagram illustrating an immunochromatographic specimen 10 according to the second embodiment.

In figure 2, the immunochromatographic specimen 10 includes a reaction layer carrier support 1, a sample addition portion 2, a marker holding portion 3, a reaction layer 4, a specific protein fixing portion 5, an absorption portion 6, and a cell component destroying substance holding portion 7.

The reaction layer carrier support 1 is composed of liquid-impermeable plastic or the like and supports chromatographic materials. The sample addition portion 2 is composed of an absorbent material such as non-woven fabric, and adds or applies a liquid sample. The marker holding portion 3 holds a marker reagent so as to dissolve into the non-woven fabric or the like. The reaction layer 4 is composed of nitrocellulose or the like. The specific protein fixing portion 5 fixes specific protein which performs binding-reaction specifically to an analyte such as antigen or antibody in accordance with the reaction mode, onto the region of the reaction layer 4. The absorption portion 6 finally absorbs the liquid sample. The cell component destroying substance holding portion 7 holds a substance which destroys cell components. The sample addition portion 2, the marker holding portion 3, the reaction layer 4, the specific protein fixing portion 5, the absorption portion 6, and the cell component destroying substance holding portion 7 as described above are respectively provided above the reaction layer carrier support 1. It is necessary to select the appropriate marker reagent, the appropriate specific protein, and the appropriate cell component destroying substance in accordance with the analyzed sample and the analyte.

Further, a substance produced by the marker is a substance which, for example, is produced by the marker in the specific protein fixing portion 5, and can be luminous. A marker after completion of reaction is a marker after having being colored, for example, by coloring reaction. A reactant by the marker is a substance after having being colored by adding a substrate to the specific protein fixing portion 5 where the marker is fixed, for example, in a case where the marker is an enzyme. A dye sol, a water-soluble pigment, and a metal sol such as a gold colloid, a non-metal sol such as selenium and carbon, an oxidized metal particle such as ferrite, a colored particle such as latex, an enzyme or the like, or a complex of these substances and a substance which performs binding reaction specifically to the analyte, are used, as the marker reagent held by the marker holding portion 3.

Further, chloride such as sodium chloride or potassium chloride, nonpolar surfactant such as a triton system and a tween system, polar surfactant, or saponins are used, as the cell component destroying substance held by the cell component destroying substance holding portion 7.

Further, a structure of the immunochromatographic specimen 10 in figure 2 is an example. Any structure in which chromatography is performed, including the cell component destroying substance holding portion 7, may be taken, and the used members may be composed of an arbitrary carrier and reagent.

A specimen comprising a chromatographic material composed of an arbitrary porous carrier, such as nitrocellulose and glassfiber filter, is used as the immunochromatographic specimen 10.

Next, the immunochromatographic specimen 10 and a chromatograph analyzing method according to the second embodiment will be described.

In figure 2, when the liquid sample is added to the sample addition portion 2, the liquid sample infiltrates into the sample addition portion 2 and reaches the region of the cell component destroying substance holding portion 7. The cell components in the liquid sample are destroyed by the function of the cell component destroying substance included in this region. The liquid sample in which these cell components are destroyed reaches the marker holding portion 3. Next, the marker reagent held in the region of the marker holding portion 3 is dissolved by infiltration of the liquid sample and infiltrates into the reaction layer 4 together with the liquid sample. There is provided the specific protein fixing portion 5 where the specific protein is fixed on the region of the reaction layer 4. When the liquid sample contains the analyte, the specific protein performs antigen-antibody reaction to the complex of the analyte and the marker reagent, and a kind of coloring reaction is observed at the region of the specific protein fixing portion 5. When the liquid sample does not contain the analyte, no antigen-antibody reaction occurs, and no coloring reaction is observed, either. Finally, the liquid sample is absorbed at the absorption portion, and thereby concluding the reaction.

After concluding the above-described reaction, a signal from the marker in the coloration region is measured by employing an arbitrary detecting instrument. When the sample is whole blood, the measurement is preferably performed, for example, at an arbitrary wavelength of 580nm or above. Thereby, the presence of coloration, and the coloration degree can be qualitatively or quantitatively analyzed. It can be detected whether or not the liquid sample contains the analyte by the above-described analysis, and, when the liquid sample contains the analyte, its quantity can be measured. Further, the "signal from the marker" described above concretely means a color of the marker which itself is a colored item, light emission by uv irradiation or voltage application, or coloration by coloring reaction.

In the measurement of the signal from the marker in the coloration region, besides a case where an integral value of an absorbance or an absorbance distribution is measured by an arbitrary wavelength, the absorbance may be measured in the wavelength region having an arbitrary width. In this case, the absorbance can be integrated in the specified wavelength region and be subjected to quantitatively analysis.

As described above, according to the immunochromatographic specimen and the chromatograph analyzing method of the second embodiment, the signal from the marker in the coloration region on the immunochromatographic specimen 10 is measured, for example, at an arbitrary wavelength of 580nm or above, thereby measuring the coloration degree at the wavelength except 200-580nm which is an absorption wavelength of blood, in analyzing colored components, particularly, the sample containing blood pigments in blood. Therefore, the qualitative or quantitative measurement of the coloration degree can be performed without being affected by the background coming from colored components included in the liquid sample, particularly, blood pigments, thereby reducing the measurement error, and realizing the qualitative or quantitative analysis by the immunochromatography with higher sensitivity and higher performance.

Further, since there is provided a portion for holding the substance which destroys the cell components, the cell components are destroyed to smooth infiltration onto the reaction layer 4 at analyzing the liquid sample containing the cell components, thereby shortening the measurement time, and performing quantitative analysis more exactly. Further, no preprocessing such as centrifugation for the sample having the cell components such as blood is necessitated, thereby realizing the lowering of cost and the improvement in the usability.

Further, a chromatograph qualitative analysis by visual observation of the colored sample can be realized in accordance with the used marker reagent.

Further, in this second embodiment, sandwich reactions having the measurement mode of the binding reaction between the marker complex and the analyte and the binding reaction between this reactant and a fixed substance is described as an example. However, the measurement can be performed even by a competition reaction in which the analyte and the marker complex competitively reacts to the fixed substance.

Further, in this second embodiment, in a case where the sample is whole blood, the signal from the marker is measured at an arbitrary wavelength of 580nm or above. However, the measurement may be preferably performed at an arbitrary wavelength from 580nm to 700nm.

Further, in the chromatograph analyzing method of the second embodiment, for example, a reflecting spectrophotometer can be used as the detecting instrument for measuring the coloration degree of the immunochromatographic specimen 10. In addition to the similar effects as the second embodiment, an opaque material can be used as the reaction layer carrier support 1 which supports the reaction layer 4. Therefore, choices of materials are increased, and further the measurement can be performed without being cautious of the rear surface state of the reaction layer 4.

Further, in the chromatograph analyzing method of the second embodiment, for example, a permeable spectrophotometer can be used as the detecting instrument for measuring the coloration degree of the immunochromatographic specimen 10. In addition to the similar effects as the second embodiment, the coloration degree of a deep portion in the reaction layer 4 can be also measured. Therefore, qualitative or quantitative analysis is possible with higher sensitivity and higher performance. Further, the marker reagent with low concentration can be used.

In this second embodiment, blood is especially used as the sample containing the colored components and the cell components. However, this is just an example, and the sample containing colored components except blood and the cell components can be analyzed. For example, body fluid, liquid containing bacteria, crushed liquid from foods or the like are also used. In this case, it is necessary to select the marker reagent and antibody in accordance with the analyzed sample and the analyte. That is, it is necessary that an absorption coefficient at the wavelength specific to the color reaction is sufficiently higher than the absorption coefficient of the sample at the same wavelength. Further, in the measurement of coloration degree by the detecting instrument, the measurement may be performed at the wavelength specific to the color reaction. Further, the "specific wavelength" described above means an arbitrary wavelength or a broad wavelength region having an arbitrary width, except a maximum wavelength region of the colored sample. Further, the "sufficiently higher" described above means that the signal from the marker by the coloring reaction is high to the extent that a reliable result of the qualitative or quantitative analysis is obtained.

### Examples

A method for implementing the present invention will be described more specifically taking the following examples. Further, those examples described hereinafter will not limit the scope of the present invention thereto.

### (Quantitation of hCG in blood)

An immunochromatographic specimen having an anti-hCG-antibody fixing line and a broad band of a complex of anti-hCG-α antibody and gold colloid in nitrocellulose membrane, is manufactured. This specimen is shown in figure 2. In the figure, the specimen includes an antibody fixing portion 5, a marker holding portion 3 where the complex of anti-hCG-α antibody and gold colloid is contained, which is disposed before the antibody fixing portion 5, and a sample addition portion 2. The manufacturing method of this specimen is as follows.

### EXAMPLE 1.

### Preparation of chromatographic specimen:

An anti-hCG-β antibody solution, the concentration of which is adjusted by dilution with phosphoric acid buffer solution, is prepared. This antibody solution is applied onto a nitrocellulose membrane employing a solution discharger. Thereby, antibody fixing line for detection is obtained on the nitrocellulose membrane. After being dehydrated, this nitrocellulose membrane is soaked in Tris-HCl buffer solution containing 1% skim milk, and is shaken gently for 30 minutes. After 30 minutes have passed, the membrane is moved to a Tris-HCl buffer solution bath and is shaken gently for 10 minutes and, thereafter, it is moved to another Tris-HCl buffer solution bath and is shaken gently for another 10 minutes, and thereafter, the nitrocellulose membrane is washed. After being washed twice, the membrane is taken out of the washing solution, and is dehydrated at room temperature.

Gold colloid is prepared in such a way that 1% citric acid solution is added to 0.01% gold chloride acid solution at 100°C while circulating. After circulated for 30 minutes, the solution is cooled. Anti-hCG-α antibody is added to the gold colloidal solution which is prepared in pH 9 by 0.2M potassium carbonate solution and then the gold colloidal solution is stirred for several minutes and, thereafter, 10% BSA (bovine serum albumin) solution of pH 9 is added in such amounts that the final concentration thereof will be 1% and then the gold colloidal solution is stirred, whereby antibody gold colloidal complex (marker antibody) is prepared. Then, this marker antibody solution is subjected to centrifugation for 50 minutes at 4°C by 20000G to isolate a marker antibody, and the marker antibody is suspended in washing buffer solution (1% BSA, phosphoric acid buffer solution), followed by centrifugation, whereby the marker antibody is washed and isolated. This marker antibody is suspended in washing buffer solution and then filtrated with a 0.8µm filter and, thereafter the marker antibody is prepared to be 1/10 of the original gold colloidal solution and then stored at 4°C.

The marker antibody solution is set in a solution discharger, and applied onto a part of the anti-hCG-β antibody fixing dehydrated membrane, which part is away from the position where the antibody is fixed, and thereafter, the membrane is dehydrated. Thereby, the marker antibody holding portion is produced on the fixed membrane.

The antibody fixing membrane including the marker antibody holding portion, which is prepared as mentioned above, is stuck onto the reaction layer carrier support, and non-woven fabric which is dehydrated after being impregnated with surfactant as hemolysis agent and glassfiber filter are added as a sample addition portion and an absorption portion, respectively. Thereafter, the membrane is cut into strips of 0.5 cm width, whereby a specimen is fabricated.

### EXAMPLE 2

### Preparation of sample:

Human blood to which heparin is added as anticoagulant is prepared so as to have a hematocrit value of 45%. By adding hCG solutions of known concentrations to this blood, hCG solutions of various known concentrations are prepared.

### EXAMPLE 3.

### Examination of wavelength at which absorbance is to be measured:

200µl or more blood containing hCG is added to the sample addition portion on specimens, and then it is developed in the direction of an absorption portion, thereby to perform an antigen-antibody reaction, and a coloring reaction in the antibody fixing portion. The state of coloration at 5 minutes having passed after adding the sample to the specimens is measured with a reflecting spectrophotometer (CS9300; Shimadzu Corporation-made), and then arithmetic processing of the coloration degree is performed.

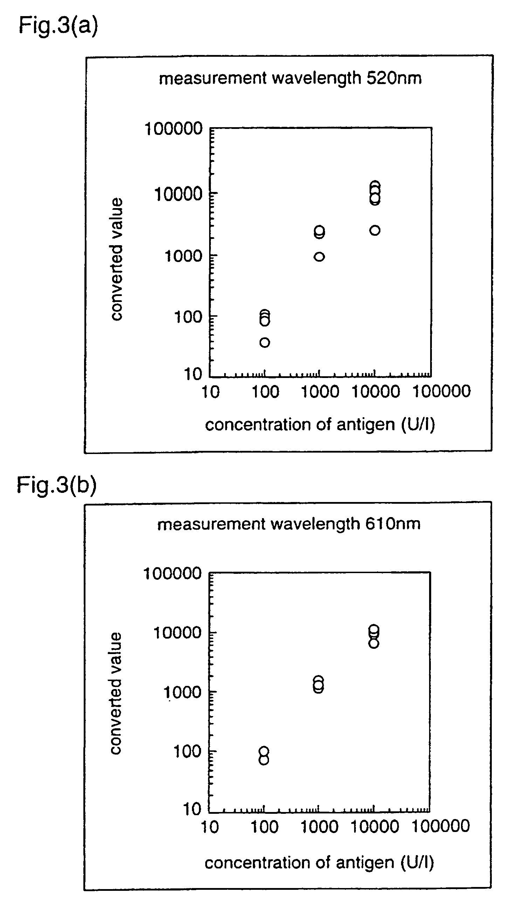

The blood (hematocrit value 45%) containing respectively 0 U/l, 100 U/l, 1000 U/l, and 10000 U/l of hCG, are added to specimens, and then developed. The states of coloration at the antibody fixing portion on the specimen to the blood of respective hCG concentrations, are measured with the reflecting spectrophotometer. Absorbances at 520nm and 610nm wavelengths are measured, and are substituted for a previously-prepared calibration curb which shows the relationship between the concentration of hCG and the absorbance. The result is shown in figure 3. Primarily, when absorbance of blood containing 1000 U/l hCG is measured and substituted for the calibration curb, the hCG concentration ought to be 1000 U/l. In practice, however, there is a slight divergence, and by the degree of that divergence, accuracy of the measurement can be known.

Figures 3 are diagrams showing quantitative performance in the case (a) of measuring absorbance in chromatographic quantitative measurement at the wavelength of 520nm and in the case (b) of measuring the same at the wavelength of 610nm. An axis of abscissas shows hCG concentration of the sample added to the specimens. An axis of ordinates shows a converted value of the antigen concentration, which value is obtained by substituting a signal from a marker in a coloration region on the specimen for the calibration curb.

Hereinafter, the effects will be described of the case where the measurement is performed at 580nm or less wavelength and the case where the measurement is performed at 580nm or more wavelength, in the chromatographic quantitative measurement.

In figure 3, converted results of the concentration of the analyte are shown on the basis of the measured values of coloration degree at 5 minutes having passed after a liquid sample is added to the immunochromatographic specimen. As the marker reagent in both cases of (a) and (b), the same antibody, gold colloid complex, is used. Initially, when the measurement is performed at 610nm (figure 3(b)), CV values (coefficients of variation) are within the range from 0% to 15%. On the other hand, when the measurement is performed at 520nm ( figure 3(a)), CV values vary from 35 to 45% by receiving affects of blood corpuscle pigments, and this large dispersion indicates that the quantitative performance is inferior.

In this embodiment, the result has been described using the gold colloid as the marker. However, the gold colloid preferably has a particle diameter having an absorption at 580nm or more wavelength. In addition, if the marker except the gold colloid is colored particles or water-soluble pigments, the measurement is desirably performed at a maximum absorption wavelength of the colored pigments, for example, around 650nm wavelength in the case of blue particles or blue pigments, or at an arbitrary 580nm or more wavelength in the case of carbon black. The measurement wavelength comes from the absorption wavelength of the colored reactant.

### Industrial Availability

In the present invention, when chromatograph analysis is performed for qualitatively or quantitatively analyzing the liquid sample by utilizing antigen-antibody reaction, the marker itself or a substance produced by the marker is made to have the wavelength region including the absorption wavelength which is different from the absorption wavelength of the sample containing the analyte, or having the same absorption wavelength including the absorption coefficient which is sufficiently higher than the absorption coefficient of the sample, thereby performing an analysis without preprocessing the colored sample such as blood.

## Claims

1. An immunochromatographic specimen used at the qualitative or quantitative analysis of an analyte included in a colored sample, wherein
a marker itself or a substance which is produced by the marker or a marker after completion of reaction or a reactant by the marker has a wavelength region including an absorption wavelength which is different from the absorption wavelength of the sample which contains the analyte, or having the same absorption wavelength including an absorption coefficient sufficiently higher than the absorption coefficient of the sample.

2. The immunochromatographic specimen defined in Claim 1 wherein,
in the case where the sample is blood, the absorption wavelength specific to coloration generated on the specimen is 580nm or above.

3. The immunochromatographic specimen defined in Claim 1 or 2 including a region for holding a substance, which destroys cell components included in the sample.

4. The immunochromatographic specimen defined in Claim 3 wherein
the substance which destroys cell components includes inorganic matter, surfactant, or saponins.

5. The immunochromatographic specimen defined in Claim 4 wherein
the inorganic matter contains chloride.

6. The immunochromatographic specimen defined in Claim 4 wherein
the surfactant contains nonpolar surfactant.

7. The immunochromatographic specimen defined in any one of Claims 1 through 6 wherein
a coloration degree is qualitatively or quantitatively analyzed with'a spectrophotometer.

8. The immunochromatographic specimen defined in any one of Claims 1 through 7 wherein
the marker includes at least one which is selected from a metal sol, an oxidized metal particle, a non-metal sol, a dye sol, a colored particle, a pigment, or an enzyme.

9. The immunochromatographic specimen defined in any one of Claims 1 through 8 wherein
the analyte contains at least one of plasma protein, bacteria, and virus.

10. A chromatograph analyzing method using the immunochromatographic specimen wherein
the colored sample containing the analyte is added to the immunochromatographic specimen, a coloration degree on the specimen is measured at an absorption wavelength specific to the coloration, and
a marker itself or a substance which is produced by the marker or a marker after completion of reaction or a reactant by the marker has a wavelength region including an absorption wavelength which is different from the absorption wavelength of the sample which contains the analyte, or having the same absorption wavelength including an absorption coefficient sufficiently higher than the absorption coefficient of the sample.

11. The chromatograph analyzing method defined in Claim 10 wherein,
in the case where the sample is blood, a signal from the marker in a coloration region is measured at a wavelength of any 580nm or above, thereby qualitatively or quantitatively analyzing the analyte.

12. The chromatograph analyzing method defined in Claim 10 or 11 wherein
the immunochromatographic specimen includes a region where a substance which destroys cell components in the sample is held.

13. The chromatograph analyzing method defined in Claim 12 wherein
the substance which destroys cell components includes inorganic matter, surfactant, or saponins.

14. The chromatograph analyzing method defined in Claim 13 wherein
the inorganic matter contains chloride.

15. The chromatograph analyzing method defined in Claim 13 wherein
the surfactant contains nonpolar surfactant.

16. The chromatograph analyzing method defined in any one of Claims 10 through 15 wherein
a coloration degree is qualitatively or quantitatively analyzed with a spectrophotometer.

17. The chromatograph analyzing method defined in any one of Claims 10 through 16 wherein
the marker includes at least one which is selected from a metal sol, an oxidized metal particle, a non-metal sol, a dye sol, a colored particle, a pigment, or an enzyme.

18. The chromatograph analyzing method defined in any one of Claims 10 through 17 wherein
the analyte contains at least one of plasma protein, bacteria, and virus.

## Patentansprüche

1. Immunchromatographisches Probestück, das bei der qualitativen oder quantitativen Analyse eines in einer gefärbten Probe enthaltenen Analyten verwendet wird, **dadurch gekennzeichnet, dass** eine Markierungssubstanz selbst oder eine Substanz, die mit der Markierungssubstanz hergestellt wird, oder eine Markierungssubstanz nach Abschluss der Reaktion oder ein Reaktionspartner mit der Markierungssubstanz einen Wellenlängenbereich hat, der eine Absorptionswellenlänge, die sich von der Absorptionswellenlänge der den Analyten enthaltenden Probe unterscheidet, aufweist oder der die gleiche Absorptionswellenlänge mit einem Absorptionskoeffizienten hat, der ausreichend höher als der Absorptionskoeffizient der Probe ist.

2. Immunchromatographisches Probestück nach Anspruch 1, **dadurch gekennzeichnet, dass** in dem Fall, dass die Probe Blut ist, die Absorptionswellenlänge, die für die an dem Probestück entstehende Färbung spezifisch ist, 580 nm oder mehr ist.

3. Immunchromatographisches Probestück nach Anspruch 1 oder 2, das einen Bereich zum Halten einer Substanz, die in der Probe enthaltene Zellteile zerstört, aufweist.

4. Immunchromatographisches Probestück nach Anspruch 3, **dadurch gekennzeichnet, dass** die Substanz, die Zellteite zerstört, einen anorganischen Stoff, einen grenzflächenaktiven Stoff oder Saponine aufweist.

5. Immunchromatographisches Probestück nach Anspruch 4, **dadurch gekennzeichnet, dass** der anorganische Stoff Chlorid enthält.

6. Immunchromatographisches nach Anspruch 4, **dadurch gekennzeichnet, dass** der grenzflächenaktive Stoff einen nichtpolaren grenzflächenaktiven Stoff enthält.

7. Immunchromatographisches Probestück nach einem der Ansprüche 1 bis 6, **dadurch gekennzeichnet, dass** ein Färbungsgrad qualitativ oder quantitativ mit einem Spektralphotometer analysiert wird.

8. Immunchromatographisches Probestück nach einem der Ansprüche 1 bis 7, **dadurch gekennzeichnet, dass** die Markierungssubstanz mindestens eine Komponente aus der Gruppe Metallsol, oxidiertes Metallteilchen, Nichtmetallsol, Farbstoffsol, gefärbtes Teilchen, Pigment oder Enzym aufweist.

9. Immunchromatographisches Probestück nach einem der Ansprüche 1 bis 8, **dadurch gekennzeichnet, dass** der Analyt mindestens Plasmaprotein, Bakterien oder Viren enthält.

10. Chromatographisches Analysenverfahren, das das immunchromatographische Probestück verwendet, **dadurch gekennzeichnet, dass**
die gefärbte Probe, die den Analyten enthält, in das immunchromatographische Probestück gegeben wird, ein Färbungsgrad an dem Probestück mit einer für die Färbung spezifischen Absorptionswellenlänge gemessen wird und
eine Markierungssubstanz selbst oder eine Substanz, die mit der Markierungssubstanz hergestellt wird, oder eine Markierungssubstanz nach Abschluss der Reaktion oder ein Reaktionspartner mit der Markierungssubstanz einen Wellenlängenbereich hat, der eine Absorptionswellenlänge, die sich von der Absorptionswellenlänge der den Analyten enthaltenden Probe unterscheidet, aufweist oder der die gleiche Absorptionswellenlänge mit einem Absorptionskoeffizienten hat, der ausreichend höher als der Absorptionskoeffizient der Probe ist.

11. Chromatographisches Analysenverfahren nach Anspruch 10, **dadurch gekennzeichnet, dass** in dem Fall, dass die Probe Blut ist, ein Signal von der Markierungssubstanz in einem Färbungsbereich bei einer Wellenlänge von 580 nm oder mehr gemessen wird, wodurch der Analyt qualitativ oder quantitativ analysiert wird.

12. Chromatographisches Analysenverfahren nach Anspruch 10 oder 11, **dadurch gekennzeichnet, dass** das immunchromatographische Probestück einen Bereich aufweist, wo eine Substanz, die Zellteile in der Probe zerstört, gehalten wird.

13. Chromatographisches Analysenverfahren nach Anspruch 12, **dadurch gekennzeichnet, dass** die Substanz, die Zellteile zerstört, einen anorganischen Stoff, einen grenzflächenaktiven Stoff oder Saponine aufweist.

14. Chromatographisches Analysenverfahren nach Anspruch 13, **dadurch gekennzeichnet, dass** der anorganische Stoff Chlorid enthält.

15. Chromatographisches Analysenverfahren nach Anspruch 13, **dadurch gekennzeichnet, dass** der grenzflächenaktive Stoff einen nichtpolaren g renzflächenaktiven Stoff enthält.

16. Chromatographisches Analysenverfahren nach einem der Ansprüche 10 bis 15, **dadurch gekennzeichnet, dass** ein Färbungsgrad qualitativ oder quantitativ mit einem Spektralphotometer analysiert wird.

17. Chromatographisches Analysenverfahren nach einem der Ansprüche 10 bis 16, **dadurch gekennzeichnet, dass** die Markierungssubstanz mindestens eine Komponente aus der Gruppe Metallsol, oxidiertes Metallteilchen, Nichtmetallsol, Farbstoffsol, gefärbtes Teilchen, Pigment oder Enzym aufweist.

18. Chromatographisches Analysenverfahren nach einem der Ansprüche 10 bis 17, **dadurch gekennzeichnet, dass** der Analyt mindestens Plasmaprotein, Bakterien oder Viren enthält.

## Revendications

1. Spécimen immunochromatographique utilisé lors de l'analyse qualitative ou quantitative d'un analyte inclus dans un échantillon coloré, dans lequel
un marqueur lui-même ou une substance qui est produite par le marqueur, ou un marqueur après achèvement d'une réaction ou bien un réactif au marqueur, présente une région de longueurs d'onde comprenant une longueur d'onde d'absorption qui est différente de la longueur d'onde d'absorption de l'échantillon qui contient l'analyte, ou présente la même longueur d'onde d'absorption avec un coefficient d'absorption suffisamment plus élevé que le coefficient d'absorption de l'échantillon.

2. Spécimen immunochromatographique selon la revendication 1, dans lequel,
dans le cas où l'échantillon est du sang, la longueur d'onde d'absorption spécifique de la coloration générée sur le spécimen est de 580 nm ou plus.

3. Spécimen immunochromatographique selon la revendication 1 ou 2, comprenant une région destinée à contenir une substance, qui détruit les composants de cellules inclus dans l'échantillon.

4. Spécimen immunochromatographique selon la revendication 3, dans lequel
la substance qui détruit des composants de cellules comprend une matière inorganique, un tensioactif ou des saponines.

5. Spécimen immunochromatographique selon la revendication 4, dans lequel
la matière inorganique contient un chlorure.

6. Spécimen immunochromatographique selon la revendication 4, dans lequel
le tensioactif contient un tensioactif non polaire.

7. Spécimen immunochromatographique selon l'une quelconque des revendications 1 à 6, dans lequel
le degré de coloration est qualitativement ou quantitativement analysé avec un spectrophotomètre.

8. Spécimen immunochromatographique selon l'une quelconque des revendications 1 à 7, dans lequel
le marqueur comprend au moins un élément qui est sélectionné parmi un sol métallique, une particule métallique oxydée, un sol non métallique, un sol de colorant, une particule colorée, un pigment ou une enzyme.

9. Spécimen immunochromatographique selon l'une quelconque des revendications 1 à 8, dans lequel
l'analyte contient au moins un élément parmi une protéine de plasma, des bactéries et un virus.

10. Procédé d'analyse par chromatographie utilisant le spécimen immunochromatographique dans lequel
l'échantillon coloré contenant l'analyte est ajouté au spécimen immunochromatographique, le degré de coloration sur le spécimen est mesuré à une longueur d'onde d'absorption spécifique à la coloration, et
le marqueur lui-même ou une substance qui est produite par le marqueur, ou bien un marqueur après achèvement de la réaction ou un réactif au marqueur, présente une région de longueurs d'onde comprenant une longueur d'onde d'absorption qui est différente de la longueur d'onde d'absorption de l'échantillon qui contient l'analyte, ou bien présente la même longueur d'onde d'absorption avec un coefficient d'absorption suffisamment plus élevé que le coefficient d'absorption de l'échantillon.

11. Procédé d'analyse par chromatographie selon la revendication 10, dans lequel,
dans le cas où l'échantillon est du sang, le signal provenant du marqueur dans une région de coloration est mesuré à une longueur d'onde quelconque de 580 nm ou plus, en analysant ainsi qualitativement ou quantitativement l'analyte.

12. Procédé d'analyse par chromatographie selon la revendication 10 ou 11, dans lequel
le spécimen immunochromatographique comprend une région où une substance qui détruit des composants de cellules dans l'échantillon est contenue.

13. Procédé d'analyse par chromatographie selon la revendication 12, dans lequel
la substance qui détruit des composants de cellules comprend une matière inorganique, un tensioactif ou des saponines.

14. Procédé d'analyse par chromatographie selon la revendication 13, dans lequel
la matière inorganique contient un chlorure.

15. Procédé d'analyse par chromatographie selon la revendication 13, dans lequel
le tensioactif contient un tensioactif non polaire.

16. Procédé d'analyse par chromatographie selon l'une quelconque des revendications 10 à 15, dans lequel
le degré de coloration est analysé qualitativement ou quantitativement avec un spectrophotomètre.

17. Procédé d'analyse par chromatographie selon l'une quelconque des revendications 10 à 16, dans lequel
le marqueur comprend au moins un élément qui est sélectionné parmi un sol métallique, une particule métallique oxydée, un sol non métallique, un sol de colorant, une partie colorée, un pigment ou une enzyme.

18. Procédé d'analyse par chromatographie selon l'une quelconque des revendications 10 à 17, dans lequel
l'analyte contient au moins un élément parmi une protéine de plasma, des bactéries et un virus.
